# EUROPEAN PATENT SPECIFICATION

(11) **EP 3 326 693 B1**
(45) Date of publication and mention of the grant of the patent: **28.08.2019**
(21) Application number: 16836914.8
(22) Date of filing: 19.07.2016
(51) Int. Cl.: A61N 5/06, A61N 5/00

(54) **PHOTOTHERAPY APPARATUS**
LICHTTHERAPIEVORRICHTUNG
APPAREIL DE PHOTOTHÉRAPIE

(30) Priority: 18.08.2015 JP 2015160821
(43) Date of publication of application: 30.05.2018
(73) Proprietor: Ushio Denki Kabushiki Kaisha, Tokyo 100-8150 (JP)
(72) Inventor: KANEDA Kazuyuki, Tokyo 100-8150 (JP); NIHOMMORI Shingo, Tokyo 100-8150 (JP); MIMA Takaomi, Tokyo 100-8150 (JP)
(74) Representative: Tomerius, Isabel
(86) International application number: PCT/JP2016/071146
(87) International publication number: WO 2017/029923

(56) References cited:
- WO-A1-2008/017975
- JP-A- 2004 242 790
- JP-A- 2004 505 734
- JP-A- 2013 166 132
- JP-U- 3 071 007
- US-A1- 2011 037 844

## Description

### TECHNICAL FIELD

The present invention relates to a phototherapy apparatus for treating human bodies with light, and in particular relates to a handy phototherapy apparatus for treating skin diseases or the like with ultraviolet light or radiation.

### BACKGROUND ART

For the purpose of treating skin disorders such as psoriasis and leukoderma, phototherapy apparatuses that treat such skin disorders by irradiating affected parts with narrow-band UVB light (wavelength of 280 nm to 320 nm) are known. There are a variety of light sources that emit UVB light, and one of such light sources is an excimer lamp. The technique disclosed in Japanese Patent No. 4670780 (Patent Literature Document 1) and so on is known, for example, as a technique pertaining to a phototherapy apparatus that includes such an excimer lamp.

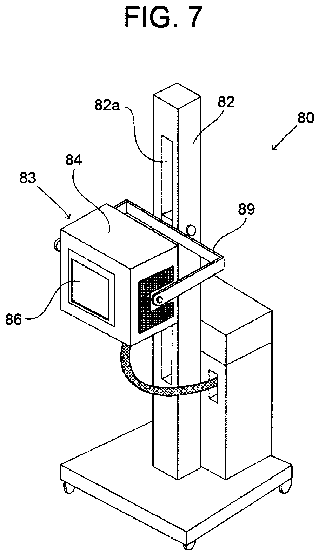

Fig. 7 of the accompanying drawings illustrates a phototherapy apparatus that is based on this existing technique.

Referring to Fig. 7, a phototherapy apparatus 80 includes a light irradiation unit 83 that is supported in a guide groove 82a in a stand 82 and that can freely move vertically therein. To be more specific, the light irradiation unit 83 is pivotally supported at a leading end of a retainer 89, and the retainer 89 is supported in the guide groove 82a in the stand 82 and can move vertically therein.

With this configuration, the light irradiation unit 83 is supported such that the light irradiation unit 83 can freely move vertically and can freely pivot. The height and the orientation of a window portion 86 in the light irradiation unit 83 can be adjusted in accordance with the location of an affected part, and the light irradiation unit 83 can irradiate the affected part with ultraviolet light or radiation under an appropriate condition.

An excimer lamp (not illustrated) is received in a housing 84 of the light irradiation unit 83, and the window portion 86 made of silica glass or the like is provided in the front surface of the housing 84.

Such a phototherapy apparatus is advantageous in that a relatively broad area of an affected part can uniformly be irradiated with light. On the other hand, the therapy apparatus itself is large and thus occupies a large installation space at a treatment site, and there is also a problem in that such a phototherapy apparatus involves a complicated operation.

To solve such an inconvenience of an installation-type therapy apparatus, a small-sized, handy phototherapy apparatus is proposed. For example, Japanese Patent Application Laid-Open Publication No. 2006-116115 (Patent Literature Document 2) discloses such phototherapy apparatus.

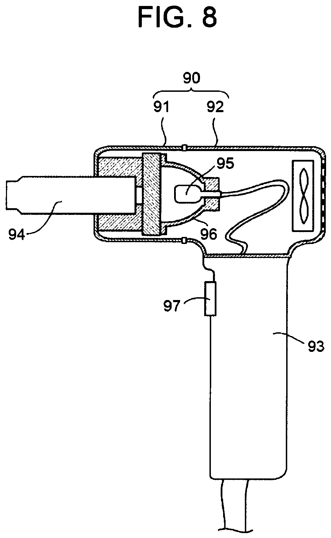

Fig. 8 of the accompanying drawings illustrates a schematic structure of the phototherapy apparatus of Patent Literature Document 2. A main case 90 of the therapy apparatus is an injection-molded component of a synthetic resin and is formed into a two-piece structure composed of a front case 91 and a back case 92. A grip 93 is integrally formed with the back case 92 at a lower portion of the back case 92, and the therapy apparatus is overall small-sized, lightweight, and handy. A light guide 94, which is a bundle of optical fibers, is removably mounted to a leading end of the front case 91. A light source unit is constituted by a filament lamp 95 and a metal reflection mirror 96. Light emitted by the filament lamp 95 is reflected by the reflection mirror 96, travels through the light guide 94, and is applied onto an affected part. An operator holds the grip 93 and presses a push switch 97 to irradiate the affected part with far-infrared light for approximately three to ten minutes with the leading end of the light guide 94 oriented toward the affected part.

This handy phototherapy apparatus utilizes far-infrared radiation of the filament lamp, and the subjects that can be treated therewith are limited. There is also a problem in that when the phototherapy apparatus irradiates an affected part to be irradiated from a short distance, the affected part is less visible and an appropriate treatment becomes difficult.

Accordingly, there is a demand for a handy phototherapy apparatus that utilizes ultraviolet radiation, as disclosed in Patent Literature Document 1, and that is small-sized, is easy to handle, and can allow an operator to perform an appropriate irradiation treatment.

Further, document US 2011 0037844 shows a handy UV emitting apparatus with an imager to visualise and analyse the treated skin.

### LISTING OF PRIOR ART REFERENCES

### Patent Literature Documents

Patent Literature Document 1: Japanese Patent No. 4670780
Patent Literature Document 2: Japanese Patent Application Laid-Open Publication No. 2006-116115

### SUMMARY OF THE INVENTION

### PROBLEMS TO BE SOLVED BY THE INVENTION

In consideration of the problems of the above-described conventional installation-type therapy apparatus, an object of the present invention is to provide a handy phototherapy apparatus that has a housing and an ultraviolet light source received in the housing, that can irradiate a target area (affected part) with ultraviolet light while observing the target area, that is easy to handle and allows an operator to perform an appropriate treatment on the affected part, that can reduce an amount of ultraviolet light leaking to the outside, and that can uniformly irradiate the affected part with the ultraviolet light at an uniform illuminance.

### SOLUTION TO THE PROBLEMS

In order to achieve the object, a phototherapy apparatus according to one aspect of the present invention includes a light guide path formed in the housing such that ultraviolet light from the ultraviolet light source passes through the light guide path. The apparatus also includes an irradiation window provided at a leading end (or a free end) of the light guide path such that the ultraviolet light exits from the irradiation window. The apparatus also includes an observation window provided in at least one side surface (side plate) of the light guide path in the vicinity of the irradiation window.

The phototherapy apparatus may further include an ultraviolet light reflection film at or over the observation window.

The ultraviolet light source may stand vertically in the housing, and the apparatus may further include an ultraviolet reflection film on an inner surface of an upper plate of the light guide path and another ultraviolet reflection film on an inner surface of a lower plate of the light guide path.

The phototherapy apparatus may further include an optical filter disposed between the irradiation window and the ultraviolet light source for reducing the ultraviolet light having a wavelength range harmful to a skin.

### ADVANTAGEOUS EFFECTS OF THE INVENTION

With the phototherapy apparatus of the present invention, the irradiation window formed at the leading end of the light guide path, through which the ultraviolet light passes, in the housing is brought into contact with or in the vicinity of an affected part, and the ultraviolet light is emitted to the affected part. Therefore, an operator can perform an extremely appropriate and effective treatment. Also, the operator can perform the irradiation treatment while watching the affected part through the observation window formed in the side surface of the light guide path near the irradiation window. Therefore, the operator can irradiate only the affected part with the ultraviolet light in a reliable manner, without erroneously irradiating other parts with the ultraviolet light.

When the ultraviolet reflection film is formed on the observation window, the ultraviolet light does not leak to the outside unnecessarily. All the ultraviolet light is directed to the affected part through the irradiation window in an efficient manner.

The ultraviolet light from the ultraviolet light source, which is arranged vertically (or stands vertically) in the housing, is reflected by the ultraviolet reflection films formed on the inner surfaces of the upper and lower plates of the light guide path, and exits through the irradiation window. Accordingly, the uniformity of the ultraviolet light illuminance in the vertical direction improves, and the illuminance of the ultraviolet light directed to the affected part increases.

The ultraviolet light that is harmful to the skin is reduced by the optical filter. Therefore, safety of the treatment is ensured.

The invention is defined in the claims; other embodiments are merely exemplary.

### BRIEF DESCRIPTION OF THE DRAWINGS

Fig. 1(A) is a front perspective view of a phototherapy apparatus according to one embodiment of the present invention, and Fig. 1(B) is a rear perspective view of the phototherapy apparatus.
Fig. 2(A) is a front view of the phototherapy apparatus according to the embodiment of the present invention, and Fig. 2(B) is a side view of the phototherapy apparatus.
Fig. 3(A) is an enlarged side view of the phototherapy apparatus according to the embodiment of the present invention, with some parts being shown in a cross-sectional view, and Fig. 3(B) is a cross-sectional view taken along the line X-X in Fig. 3(A).
Fig. 4(A) is a front view of a light irradiation unit of the phototherapy apparatus according to the embodiment of the present invention, Fig. 4(B) is a side cross-sectional view of the phototherapy apparatus, and Fig. 4(C) is a top cross-sectional view of the phototherapy apparatus.
Fig. 5 is a set of views useful to describe advantages of the light irradiation unit according to the embodiment of the present invention, when viewed in a lateral direction.
Fig. 6 is a set of views useful to describe advantages of the light irradiation unit according to the embodiment of the present invention, when viewed from above.
Fig. 7 is a perspective view of a conventional phototherapy apparatus.
Fig. 8 shows a conventional handy phototherapy apparatus.

### DETAILED DESCRIPTION OF EMBODIMENTS

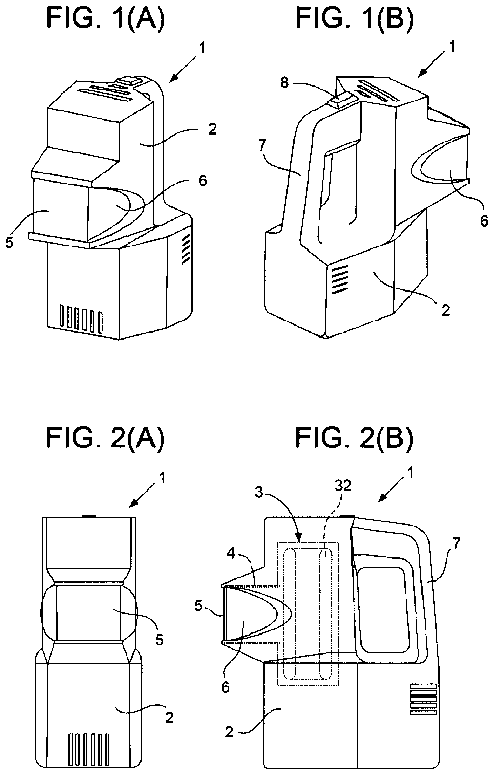

Fig. 1 is a set of perspective views illustrating a configuration of a phototherapy apparatus according to an embodiment of the present invention. Specifically, Fig. 1(A) is a perspective view as viewed from the front right, and Fig. 1(B) is a perspective view as viewed from the back right. Fig. 2(A) is a front view of the phototherapy apparatus, and Fig. 2(B) is a side view of the phototherapy apparatus.

A phototherapy apparatus 1 includes a housing 2, and a light irradiation unit 3 that has an ultraviolet lamp 32 serving as an ultraviolet light source (ultraviolet radiation source) received in the housing 2. A light guide path 4 is provided in a front surface of the light irradiation unit 3. An irradiation window 5, which transmits ultraviolet light (ultraviolet radiation), is provided at a leading end of the light guide path 4. The irradiation window 5 is made from silica glass or the like. Observation windows 6 are formed in side surfaces at the leading end of the light guide path 4.

A handle 7 is provided in a back portion of the housing 2. An operator (doctor) holds the phototherapy apparatus 1 by the handle 7 and irradiates an affected part of a patient with ultraviolet radiation from the ultraviolet lamp 32 in the light irradiation unit 3 with the irradiation window 5 in the front surface of the phototherapy apparatus being in contact with or in the vicinity of the affected part. At this point in time, the operator can irradiate the affected part with ultraviolet light while observing the affected part through the observation windows 6 in the side surfaces of the light guide path 4.

A switch 8 is provided on an upper portion of the handle 7.

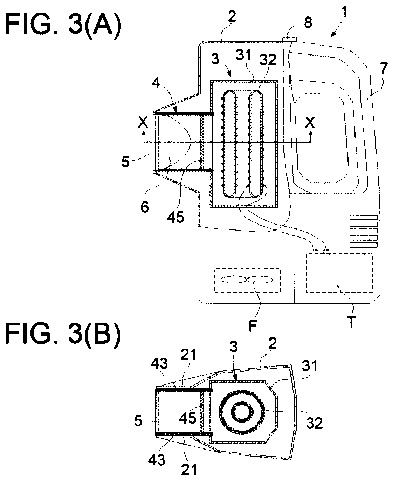

Fig. 3(A) illustrates a side view with some portions of the phototherapy apparatus 1 shown in a cross-sectional view, and Fig. 3(B) illustrates a cross-sectional view taken along the X-X line in Fig. 3(A). The light irradiation unit 3 is placed inside the housing 2, and the light irradiation unit 3 includes a lamp house 31, the ultraviolet lamp 32 placed inside the lamp house 31, and the light guide path 4 provided in a front surface of the lamp house 31.

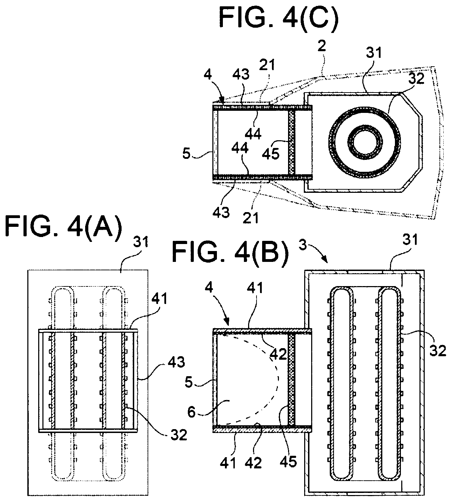

Fig. 4 is a set of views to illustrate the details of the light irradiation unit 3. Specifically, Fig. 4(A) is a front view, Fig. 4(B) is a lateral cross-sectional view, and Fig. 4(C) is a top cross-sectional view. The house 2 is supplementarily indicated by dotted lines.

The lamp house 31 is formed, for example, of metal, such as aluminum. The back and side inner surfaces, in particular, of the lamp house 31 are subjected to mirror finishing or the like, and thus high-reflection characteristics are imparted. It should be noted that the mirror finishing may not be applied to the back and side inner surfaces, and a concave reflection mirror may be disposed behind the ultraviolet lamp 32.

The ultraviolet lamp 32 is supported upright by an appropriate means within the lamp house 31. As illustrated in Fig. 3, the ultraviolet lamp 32 is electrically connected to a high-voltage transformer T provided inside the housing 2 such that the ultraviolet lamp can receive power and emit light.

The ultraviolet lamp 32 may be constituted by a low-pressure mercury lamp, an excimer lamp, or the like. In the illustrated embodiment, the ultraviolet lamp 32 is constituted by an excimer lamp. When an excimer lamp is used, the ultraviolet lamp 32 emits light at wavelength of 222 nm from a krypton-chlorine mixture gas, emits light at wavelength of 283 nm from a xenon-bromine mixture gas, emits light at wavelength of 308 nm from a xenon-chlorine mixture gas, or the like.

The light guide path 4 is provided at the front surface side of the lamp house 31. In this embodiment, the light guide path 4 has a rectangular shape, when viewed in a cross-section view, and includes upper and lower plates 41 and 41 and two side plates 43 and 43.

The upper and lower plates 41 are made of metal, such as aluminum, and an ultraviolet radiation reflection film 42 is formed on an inner surface of each of the upper and lower plates 41. The two side plates 43 are made of light-transmissive glass or the like, and an ultraviolet radiation reflection film 44 is formed on an inner surface of each of the side plates 43. The irradiation window 5 made of silica glass or the like that transmits ultraviolet radiation is formed at the leading end of the light guide path 4.

Openings 21 are formed in the housing 2 at portions toward the leading ends of the side surfaces of the light guide path 4 and in the vicinity of the irradiation window 5 (Fig. 3(B) and Fig. 4(C)), and the portions of the light-transmissive side plates 43 that correspond to the openings 21 constitute the observation windows 6. When an operator performs a treatment, the operator irradiates an affected part to be treated with ultraviolet radiation while observing the affected part through the observation windows 6 in the light-transmissive side plates 43 via the openings 21.

In the above-described structure, the openings 21 are formed in the housing 2, and portions of the light-transmissive side plates 43 are used as the observation windows 6. Alternatively, at least a leading end portion of the light guide path 4 may project from the housing 2, and the light-transmissive side plates 43 in the projecting portion of the light guide path 4 may constitute the observation windows 6.

It should be noted that the entirety of the side plates 43 may not be light-transmissive, i.e., it suffices that a region that constitutes the observation windows 6 may only be light-transmissive. However, from a fabrication standpoint, it is preferred that the entirety of the side plates 43 is made of a light-transmissive material.

The observation window 6 may be provided in only one of the side surfaces of the light guide path 4. Yet, it is desirable that the observation windows 6 be preferably provided in both side surfaces, which allows an operator to observe through both sides (or any one of the two sides) and to perform an appropriate treatment.

The light guide path 4 also has an optical filter 45 that reduces ultraviolet radiation with a short wavelength of no more than around 295 nm, which is harmful to skins. The optical filter 45 may not be disposed in the light guide path 4 but may be disposed inside the housing 2, i.e., it suffices that the optical filter 45 be disposed between the ultraviolet lamp 32 and the irradiation window 5.

Furthermore, a cooling fan F is provided inside the housing 2 to cool the ultraviolet lamp 32 in the lamp house 31.

When using the phototherapy apparatus 1, an operator holds the handle 7 and brings the irradiation window 5 into contact with an affected part. At this point in time, the operator can check and confirm the affected part, or a portion to be irradiated, through the observation windows 6 and the irradiation window 5. Upon confirming that the irradiation window 5 is appropriately situated on the affected part, the operator presses the switch 8. Then, a high voltage is applied to the ultraviolet lamp 32 from the high-voltage transformer T, a discharge starts, and ultraviolet radiation (ultraviolet light) is generated. According to a preferred embodiment, simultaneously as the switch 8 is pressed, a timer embedded therein starts operating. When a predetermined time set in advance has elapsed, the high voltage stops being applied to the ultraviolet lamp 32, and the ultraviolet lamp 32 is turned off.

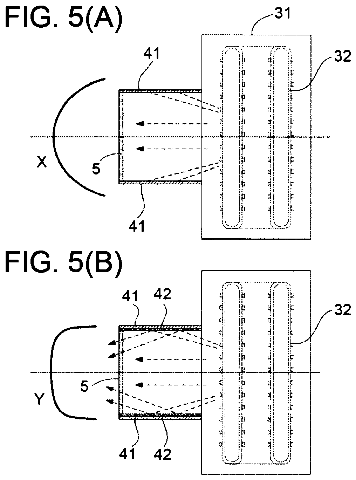

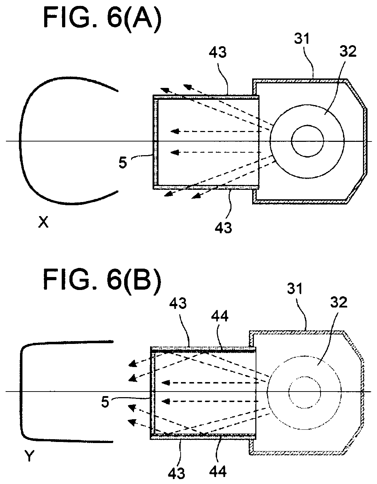

Referring now to Figs. 5 and 6, advantageous effects obtained by forming the ultraviolet radiation reflection films 42 and the ultraviolet radiation reflection films 44 on the upper and lower plates 41 and the light-transmissive side plates 43 of the light guide path 4 will be described.

Fig. 5(A) illustrates a case in which the ultraviolet lamp 32 is supported upright and no ultraviolet radiation reflection film is provided on the upper and lower plates 41 of the light guide path 4. In this case, ultraviolet radiation that hits the upper and lower plates 41 is not reflected. Thus, an ultraviolet radiation illuminance distribution X on the affected part is higher at a center portion and lower at upper and lower edge portions. Accordingly, the illuminance uniformity is not very high.

In contrast, in a case illustrated in Fig. 5(B), the ultraviolet radiation reflection films 42 are formed on the upper and lower plates 41. In this case, ultraviolet radiation that hits the ultraviolet radiation reflection films 42 is reflected by the reflection films 42, and the reflected ultraviolet radiation merges into the flux of ultraviolet radiation traveling toward the affected part and exits through the exit window 5 in the front. Thus, an illuminance distribution Y, in the vertical direction (upright direction), of the ultraviolet radiation applied onto the affected part becomes uniform, and the illuminance of the ultraviolet radiation applied onto the affected part increases.

Fig. 6(A) illustrates a case in which no ultraviolet radiation reflection film is provided on the light-transmissive side plates 43 that constitute the observation windows 6 in the light guide path 4. In this case, ultraviolet radiation that hits the side plates 43 is transmitted through the side plates 43. Thus, an ultraviolet radiation illuminance distribution X on the affected part is higher at a center portion and lower at right and left portions, and the illuminance uniformity is not very high. Furthermore, a problem may arise in which an operator (doctor) might be irradiated with the ultraviolet radiation that has leaked through the side plates 43 (observation windows 6).

In contrast, in a case illustrated in Fig. 6(B), the ultraviolet radiation reflection films 44 are formed on the light-transmissive side plates 43. In this case, ultraviolet radiation that hits the ultraviolet radiation reflection films 44 is reflected by the reflection films 44, and the reflected ultraviolet radiation merges into the flux of ultraviolet radiation traveling toward the affected part and exits through the exit window 5 in the front. Thus, an illuminance distribution Y, in the right-left direction, of the ultraviolet radiation applied onto the affected part becomes uniform, and the illuminance of the ultraviolet radiation applied onto the affected part increases.

Furthermore, because ultraviolet radiation does not leak through the observation windows 6 and is not emitted to the outside, an operator is not irradiated with ultraviolet radiation.

In the foregoing descriptions, an example in which an ultraviolet lamp is used as an ultraviolet radiation source is described. Aside from the ultraviolet lamp, an LED array can also be used as the ultraviolet radiation source, and still similar advantageous effects can be obtained.

As described above, the embodiment of the present invention provides a handy phototherapy apparatus in which an ultraviolet radiation source (ultraviolet light source) is housed inside the housing. The housing has a light guide path through which ultraviolet radiation from the ultraviolet radiation source passes. An irradiation window through which the ultraviolet radiation is applied to an affected part is provided at a leading end of the light guide path, and an observation window is provided in at least one side surface (side plate) of the light guide path at a portion in the vicinity of the irradiation window. Thus, when an operator holding the therapy apparatus in his/her hand brings the irradiation window at the leading end of the therapy apparatus into contact with or into proximity to an affected part in order to treat the affected part, the operator can do so while checking the affected part through the observation window in the side surface of the light guide path. Therefore, the operator can confirm that the irradiation window is appropriately located on the affected part and conduct an appropriate treatment.

An ultraviolet radiation reflection film is provided on (over) the light-transmissive observation window. Thus, it is possible to prevent leakage of the ultraviolet radiation, and an operator would not be irradiated with ultraviolet radiation. An illuminance distribution of ultraviolet radiation that exits through the exit window and is applied onto the affected part can be made uniform. This enhances (increases) the illuminance of the ultraviolet radiation applied onto the affected part.

Ultraviolet radiation reflection films are provided on upper and lower plates. Thus, the illuminance can be made uniform in the vertical direction when ultraviolet radiation from the vertically standing ultraviolet radiation source exits through the exit window and is applied onto the affected part.

An optical filter is provided between the irradiation window and the ultraviolet radiation source. Thus, ultraviolet radiation in a wavelength range harmful to skins can be reduced, and a safe treatment can be provided.

### REFERENCE NUMERALS AND SYMBOLS

- 1: Phototherapy apparatus
- 2: Housing
- 3: Light irradiation unit
- 31: Lamp house
- 32: Ultraviolet lamp
- 4: Light guide path
- 41: Upper and lower plates
- 42: Ultraviolet radiation reflection film
- 43: (Light-transmissive) side plate
- 44: ultraviolet radiation reflection film
- 45: Optical filter
- 5: Irradiation window
- 6: Observation window
- 7: Handle
- 8: Switch
- T: High-voltage transformer
- F: Cooling fan

## Claims

1. A handy phototherapy apparatus (1) comprising:
a housing (2);
an ultraviolet light source (32) received in the housing;
a light guide path (4) formed in the housing such that ultraviolet light from the ultraviolet light source passes through the light guide path;
an irradiation window (5) provided at a leading end of the light guide path such that the ultraviolet light exits from the irradiation window;
**characterised by** further comprising:
an observation window (6) provided in at least one of side surfaces of the light guide path in the vicinity of the irradiation window.

2. The handy phototherapy apparatus according to claim 1 further comprising an ultraviolet light reflection film (42) over the observation window.

3. The handy phototherapy apparatus according to claim 1 or 2, wherein the ultraviolet light source (32) stands vertically in the housing (1), and
the apparatus further comprises an ultraviolet reflection film on an inner surface of an upper plate of the light guide path and another ultraviolet reflection film on an inner surface of a lower plate of the light guide path.

4. The handy phototherapy apparatus according to any one of claims 1 to 3 further comprising an optical filter (45) disposed between the irradiation window and the ultraviolet light source for reducing the ultraviolet light having a wavelength range harmful to a skin.

## Patentansprüche

1. Lichttherapie-Handvorrichtung (1), umfassend:
ein Gehäuse (2),
eine in dem Gehäuse aufgenommene Ultraviolett-Lichtquelle (32),
einen Lichtleitweg (4), der in dem Gehäuse derart gebildet ist, dass ultraviolettes Licht aus der Ultraviolett-Lichtquelle den Lichtleitweg durchläuft,
ein Bestrahlungsfenster (5), das an einem vorderen Ende des Lichtleitwegs derart bereitgestellt ist, dass das ultraviolette Licht aus dem Bestrahlungsfenster austritt,
**dadurch gekennzeichnet, dass** sie ferner umfasst:
ein Sichtfenster (6), das in mindestens einer der Seitenflächen des Lichtleitwegs in der Nähe des Bestrahlungsfensters bereitgestellt ist.

2. Lichttherapie-Handvorrichtung nach Anspruch 1, ferner umfassend einen über das Sichtfenster verlaufenden Reflexionsfilm (42) für ultraviolettes Licht.

3. Lichttherapie-Handvorrichtung nach Anspruch 1 oder 2, wobei die Ultraviolett-Lichtquelle (32) vertikal in dem Gehäuse (1) steht und
die Vorrichtung ferner einen Ultraviolett-Reflexionsfilm auf einer Innenfläche einer oberen Platte des Lichtleitwegs und einen weiteren Ultraviolett-Reflexionsfilm auf einer Innenfläche einer unteren Platte des Lichtleitwegs umfasst.

4. Lichttherapie-Handvorrichtung nach einem der Ansprüche 1 bis 3, ferner umfassend ein optisches Filter (45), das zwischen dem Bestrahlungsfenster und der Ultraviolett-Lichtquelle angeordnet ist, um das ultraviolette Licht mit einem für Haut schädlichen Wellenlängenbereich zu verringern.

## Revendications

1. Dispositif de photothérapie ergonomique (1) comprenant :
un boîtier (2) ;
une source de lumière ultraviolette (32) reçue dans le boîtier ;
un trajet (4) de guidage de la lumière formé dans le boîtier de telle sorte que la lumière ultraviolette provenant de la source de lumière ultraviolette passe par le trajet de guidage de la lumière ;
une fenêtre (5) d'irradiation présente à une extrémité avant du trajet de guidage de la lumière de telle sorte que la lumière ultraviolette ressorte de la fenêtre d'irradiation ;
***caractérisé en ce qu***'il comprend en outre :
une fenêtre d'observation (6) ménagée dans au moins une des surfaces latérales du trajet de guidage de la lumière se trouvant au voisinage de la fenêtre d'irradiation.

2. Dispositif de photothérapie ergonomique selon la revendication 1, comprenant en outre un film (42) réfléchissant la lumière ultraviolette sur la fenêtre d'observation.

3. Dispositif de photothérapie ergonomique selon la revendication 1 ou 2, dans lequel la source (32) de lumière ultraviolette se dresse verticalement dans le boîtier (1), et
le dispositif comprend en outre un film réfléchissant la lumière ultraviolette sur une surface intérieure d'une plaque supérieure du trajet de guidage de la lumière et un autre film réfléchissant la lumière ultraviolette sur une surface intérieure d'une plaque inférieure du trajet de guidage de la lumière.

4. Dispositif de photothérapie ergonomique selon l'une quelconque des revendications 1 à 3, comprenant en outre un filtre optique (45) disposé entre la fenêtre d'irradiation et la source de lumière ultraviolette pour réduire la lumière ultraviolette ayant une gamme de longueurs d'onde nocive pour la peau.
